Europäisches Patentamt

European Patent Office

Office européen des brevets

(19)

(11) Veröffentlichungsnummer: **0 520 202 A2**

## (12) EUROPÄISCHE PATENTANMELDUNG

(21) Anmeldenummer: **92109022.1**

(22) Anmeldetag: **29.05.92**

(51) Int. Cl.5: **G01N 33/558**, G01N 33/58,
//G01N33/00

(30) Priorität: **28.06.91 DE 4121493**

(43) Veröffentlichungstag der Anmeldung:
**30.12.92 Patentblatt 92/53**

(84) Benannte Vertragsstaaten:
**DE FR GB NL**

(71) Anmelder: **Drägerwerk Aktiengesellschaft**
**Moislinger Allee 53-55**
**W-2400 Lübeck 1(DE)**

(72) Erfinder: **Scholtissek, Stephan, Dr.**
**Birkenstrasse 101**
**W-4000 Düsseldorf(DE)**
Erfinder: **Manns, Andreas, Dr.**
**Musterbahn 5a**
**W-2400 Lübeck 1(DE)**
Erfinder: **Rindt, Klaus-Peter, Dr.**
**Brömbsenstrasse 21**
**W-2400 Lübeck(DE)**
Erfinder: **Sohège, Jürgen, Dr.**
**Falkenstrasse 28**
**W-2400 Lübeck(DE)**
Erfinder: **Evers, Wolfgang**
**Koggenweg 3**
**W-2400 Lübeck 1(DE)**
Erfinder: **Rabenecker, Horst**
**Steenkoppel 1**
**W-2407 Klein Parin(DE)**

(54) **Analysevorrichtung zur quantitativen, immunologischen Bestimmung von Schadstoffen.**

(57) Eine Vorrichtung zur quantitativen Analyse von Schadstoffen oder schadstoffhaltigen Aerosolen mit Hilfe einer immunologischen Reaktion, bei der der nachzuweisende Schadstoff (Analyt) mit einem Antikörper in Zusammenwirkung mit einem markierten Analyten (Tracer) zu einer immunologischen Reaktion gebracht wird, soll derart verbessert werden, daß eine einfache, direkt anzeigende und auswertbare Vorrichtung geschaffen wird, die die sehr hohe Spezifität der Reaktion eines dafür spezifischen Bindepaares zur Messung von Luft-, Wasser- und Bodenproben sowie schadstoffhaltigen Aerosolen nutzbar macht. Dazu ist vorgesehen, daß die Antikörper (5) zusammen mit den markierten Tracern (7) in einem dem Analyten ausgesetzten und für diesen permeablen, undurchsichtigen Teilbereich des Trägers (100) vorliegen, durch welche die nach erfolgter immunologischer Reaktion in dem undurchsichtigen Teilbereich verbleibenden freien, nicht an einen Antikörper gebundenen Tracer in einen transparenten Teilbereich des Trägers diffundieren, so daß das Fortschreiten der Tracerdiffusion an der Außenfläche des transparenten Teilbereichs des Trägers durch dessen Verfärbung erkennbar ist.

Fig. 1

Die Erfindung betrifft eine Vorrichtung zur quantitativen Analyse von Schadstoffen oder schadstoffhaltigen Aerosolen mit Hilfe einer immunologischen Reaktion, bei der der nachzuweisende Schadstoff (Analyt) mit einem Antikörper in Zusammenwirkung mit einem markierten Analyten (Tracer) zu einer immunologischen Reaktion gebracht wird.

Zur Messung von Analyten in der Luft sind einige direktanzeigende Dosimeter beschrieben, die den zu messenden Analyten in einer chemischen Nachweisreaktion unter Bildung von Farbstoffen umsetzen. So beschreibt die US-4 272 480 ein Dosimeter für die chemische Messung von Ethylenoxid in der Luft. Das Ethylenoxid gelangt von der der Luft zugewandten und offenen Seite her an die chemische Nachweisschicht. Ethylenoxid färbt diese um so intensiver, in je höherer Konzentration es vorliegt. Die entstandene Farbintensität wird zum Maß für die Ethylenoxid-Konzentration genommen. Durch ein Fenster auf der Rückseite der Plakette ist mittels Farbvergleich die vorliegende Ethylenoxid-Konzentration ablesbar. Ein Nachteil dieser chemischen Reaktion ist darin zu sehen, daß sie eine nicht sehr große Spezifität für den Analyten besitzt. Dadurch ist die Querempfindlichkeit zu anderen Schadstoffen unerwünscht hoch.

In der EP-374 685 wird die Verwendung hochspezifischer Enzyme zum Nachweis von Formaldehyd in der Luft beschrieben. Auch hier gelangt der Analyt von der offenen Seite her in das plakettenförmige Dosimeter. Der Analyt wird in der hochspezifischen Enzymreaktion umgesetzt. In einer nachgeschalteten Redox-Reaktion entsteht umsomehr Farbstoff, je mehr Analyt in die Plakette eindringen konnte. Die Auswertung erfolgt auch hier durch einen Farbvergleich.

Im Zusammenhang mit Messungen in der Gasphase können Enzyme nur relativ kleine Moleküle von etwa bis 100 g pro mol hochspezifisch umsetzen. Eine Meßaufgabe besteht jedoch auch für Analyte mit höheren Molgewichten, wie Pflanzenschutzmittel, Sprengstoffe und Drogen, aber auch Mikroorganismen wie Viren und Bakterien oder Allergene.

Zum Nachweis solcher Analyte mit höheren Molgewichten in wäßrigen Proben verwendet vor allem die medizinsche Analytik den sogenannten Immunoassay. So beschreibt die EP-51 183 ein mehrschichtiges Analyseelement, das mindestens aus einer Trägerschicht besteht, an die für die Spezifität der Reaktion notwendigen Antikörper kovalent immobilisiert sind und mindestens eine Reagenzschicht, die die für die Farbentwicklung notwendigen Reagenzien trägt. Zur Durchführung der Analyse wird die Probe, die den Analyt enthält, mit einer definierten Menge eines mit einem Tracer (hierunter soll ein Analyt oder Analytderivat verstanden werden, der kovalent an ein oder mehrere Markierungselemente gebunden ist) gemischt. Anschließend wird das Gemisch, und nicht die wäßrige Probe selbst, auf das Analyseelement aufgetragen. Im Analyseelement läuft ein kompetitiver Immunoassay ab. Tracer, die wegen der Anwesenheit von Analyt nicht an die immobilisierten Antikörper binden können, diffundieren in die Reagenzschicht und leiten dort mittels ihres Enzymanteils die der Quantifizierung dienende enzymatische Farbreaktion ein.

In der DE-Z: "Journal of Immunological Methods", 135, 1990, 191-197, Kusterbeck et al., wird ein Immunoassay-Prinzip beschrieben, das unter dem Namen Verdrängungsimmunoassay bekannt ist. Mit diesem Verdrängungsimmunoassay entfällt ein Mischen der Probe mit dem Tracer und es kann auf eine Reagenzschicht samt den Reagenzien verzichtet werden. Diese Verdrängung läuft in einer Reaktionssäule einer Fließinjektionsanordnung ab. Bei der Messung verdrängt der Analyt den mit Fluorophoren markierten Analyt (Tracer) aus seiner Bindung mit dem spezifischen Antikörper, der an eine Matrix der Reaktionssäule gebunden ist. Da man bei dem bekannten Assay auf Reagenzien verzichten will, muß man zur Quantifizierung der Menge der verdrängten Tracer ein Fluoreszenzmeßgerät einsetzen. Da pro Analyt genau ein Tracer verdrängt wird, existiert beim Verdrängungsimmunoassay ein linearer Zusammenhang zwischen Analytkonzentration und Meßsignal. Ein Immunoassay, der mit Fluorophoren als Markierungselement arbeitet, ermöglicht zwar eine quantitative Reaktionsaussage, erfordert aber aufwendige Meßmittel.

In den meisten Fällen ist es jedoch notwendig, eine schnelle, wenn auch vorläufige, quantitative Aussage über das Vorhandensein von Art und Menge der zu untersuchenden Schadstoffe zu gewinnen.

Der vorliegenden Erfindung liegt somit die Aufgabe zugrunde, eine einfache, direkt anzeigende und auswertbare Vorrichtung der genannten Art zu entwickeln, das die sehr hohe Spezifität der Reaktion eines dafür spezifischen Bindepaares zur Messung von Luft-, wasser- und Bodenproben sowie schadstoffhaltigen Aerosolen nutzbar macht.

Die Lösung der Aufgabe erfolgt dadurch, daß die Antikörper zusammen mit den markierten Tracern in einem dem Analyten ausgesetzten und für diesen permeablen, undurchsichtigen Teilbereich des Trägers vorliegen, durch welche die nach erfolgter immunologischer Reaktion in dem undurchsichtigen Teilbereich verbleibenden freien, nicht an einen Antikörper gebundenen Tracer in einen transparenten Teilbereich des Trägers diffundieren, so daß das Fortschreiten der Tracerdiffusion an der Außenfläche des transparenten Teilbereichs des Trägers durch dessen Verfärbung erkennbar ist.

Mit der Erfindung wird der Vorteil erzielt, daß die auf dem Träger immobilisierten Antikörper infolge der immunologischen Reaktion (z.B. Konkurrenzreaktion oder Verdrängungsreaktion) einen Teil der vorliegenden Tracer an sich binden, einen anderen Teil davon nicht. Dieser freie Teil an Tracern diffundiert durch den Träger wegen des vorhandenen Konzentrationsgefälles in bezug auf die Tracer-Konzentration im Träger, bis er an der Oberfläche des sichtbaren Teilbereichs des Trägers erkennbar wird. Antikörper und Tracer sind auf der dem nachzuweisenden Schadstoff ausgesetzten Oberfläche des Trägers immobilisiert. Als Beispiel für einen membranartigen Träger kann die unter dem Namen Immunodyne-Membran vertriebene der Pall Corporation genannt werden. Diese Membran ermöglicht eine direkte kovalente Bindung von Antikörpern, wodurch sie auf der Membran irreversibel immobilisiert sind. Weitere Membranen sind aus der EP-51 183 bekannt. Die Immobilisierung von Antikörpern auf diesen Membranen durch Adsorption und/ oder Ausbildung hydrophober/hydrophiler Wechselwirkungen ist ebenfalls möglich. Die Poren der genannten Membranen sind so groß, daß sie keine oder nur eine geringe Diffusionsbarriere für die markierten Tracer darstellen. Darüber hinaus richtet sich die Porengröße nach der Größe der Analyten. Die Kopplung des Analyten an einen farbigen Kunststoff als Beispiel für eine Markierung wird in der Veröffentlichung: "Journal of Cell Biology", 64, 75-88, Molday et al., aus dem Jahre 1975 beschrieben.

Für die passive, diffusionsbedingte Messung gasförmiger Proben müssen diese Membranen mit der für die Reaktion erforderlichen Feuchte versehen werden. Dies kann durch Einbau oder Auftragen eines Feuchtespeichers erfolgen, oder durch einen mit Wasser getränkten Docht, oder durch Benetzen bzw. Aufsprühen eines Wassernebels. Die Gasprobe dringt anschließend per Diffusion in den Träger ein.

Für die aktive Probenahme mittels Durchspülung oder Durchsaugung der Probe durch den Träger wird eine Rehydratisierung von Antikörper und Tracer bewirkt, indem eine gepufferte wäßrige oder wäßrig/organische Lösung dafür verwendet wird.

Die erfindungsgemäße Vorrichtung ist durch die Kombination der immunologischen Nachweisreaktion auf der Basis eines Verdrängungsassays in Verbindung mit der Kopplung des Antikörpers an einen Tracer zu einem Tracer/ Antikörper-Komplex geeignet, zu einer einfachen, direkt auswertbaren quantitativen Analysevorrichtung eingesetzt zu werden, wodurch eine unmittelbare Überwachung der Umgebung zum Beispiel am Arbeitsplatz möglich wird, ohne daß aufwendige naßchemische Reaktionen mit unterschiedlichen Reagenzien durchgeführt, oder aufwendige Nachweisgeräte, wie z.B. Fluoreszenz-Meßgerät, für die nachträgliche quantitative Auswertung eingesetzt werden müssen. Auf eine zusätzliche Reaktionsschicht, in der die verdrängten Tracer eine Farbreaktion mit einem Reaktionspartner eingehen, kann verzichtet werden. Die verdrängten Tracer können nach erfolgter Reaktion als ungebundene, freie Tracer in die Nachweisschicht diffundieren.

Ebenso ist die erfindungsgemäße Vorrichtung geeignet, als immunologische Nachweisreaktion eine Konkurrenzreaktion zwischen den Tracern und den Analyten ablaufen zu lassen. Durch Befeuchtung z.B. mittels einer gepufferten Lösung, in der sich der zu untersuchende Schadstoff befindet, findet eine Rehydratisierung der Tracer statt, wodurch sowohl der Analyt als auch der Tracer um einen freien Bindungsplatz am Antikörper konkurrieren. Die dabei ohne Bindungsplatz bleibenden Tracer sind frei, und in der Lage, in die Nachweisschicht zu diffundieren.

Eine besonders geschickte Ausbildungsform besteht darin, daß der Träger zwei Schichten aufweist, von denen die eine als Reaktionsschicht mit den an dieser immobilisierten Tracern dem zu untersuchenden Analyten ausgesetzt ist, an welche die andere, für die freien, nicht an einen Antikörper gebundenen Tracer permeable Nachweisschicht im Diffusionskontakt anliegt. Der Ausdruck "Schicht" soll in diesem Zusammenhang so verstanden werden, daß hierfür auch eine Einzelmembran gemeint ist, deren unterschiedlichen Schichtbereiche die gewünschte Funktion erfüllen. So kann die Nachweisschicht in der dem Analyten abgewandten, besonders strukturierten Außenfläche einer Membran bestehen, wobei die Reaktionsschicht durch den Membrankörper gebildet ist. Mit einer derartigen Ausbildungsform gewinnt man den Vorteil, daß die Reaktionsschicht einerseits in bezug auf die Immobilisierung des Tracers optimiert werden kann, und die Nachweisschicht andererseits als eine für die Diffusion des Tracers geeignete Konfiguration besitzt. Dadurch ist es möglich, eine besonders günstige Farbbeobachtung zu verwirklichen. Die Auswertung der Nachweisschicht erfolgt zweckmäßigerweise mit Hilfe von Farbvergleichstandards.

Um die Nachweisempfindlichkeit der Vorrichtung zu erhöhen, ist es günstig, mehrere Markierungselemente an einen Analyten zu binden. Dies geschieht dadurch, den Analyten, oder geeignete Derivate (darunter werden mit Kopplungsgruppen versehene Analyten verstanden), erst mit einem Polymer zu versehen, welches eine repititive Sequenz von geeigneten Kopplungsgruppen enthält. Danach wird ein Markierungselement je Kopplungsgruppe, also mehrere Elemente pro Analyt oder Derivat, gebunden. Je größer somit die Zahl der Markierungselemente ist, desto höher wird die Empfindlichkeit der Nachweisvorrichtung sein. Zweckmäßigerweise werden als Markierungselemente mit dem bloßen Auge sichtbare Farbstoffmoleküle an den Analyten gebunden.

Zur Erhöhung der Ansprechgeschwindigkeit und zur Verbesserung der Diffusionseigenschaften ist es vorteilhaft, die beiden Schichten so zu gestalten, daß sie eine insgesamt voneinander verschiedene elektrische Ladung oder Polarität aufweisen. Wenn die Reaktionsschicht z.B. hydrophil ist, sind die Tracer dann in ihrer eigenen Polarität so auszubilden, daß sie eine Affinität zu der Nachweisschicht besitzen. Wenn z.B. die Tracer hydrophob sind, und die Nachweisschicht ebenfalls hydrophob ist, dann werden die Tracer infolge ihrer Hydrophobizität bevorzugt in Richtung Nachweisschicht diffundieren. Dadurch sind Querdiffusionen bzw. störende Diffusionswechselwirkungen der Tracer untereinander verringert. Man erzeugt auf diese weise einen erhöhten Diffusionsgradienten für die verdrängten Tracer in Richtung der Nachweisschicht.

Im einfachsten Fall wird der flächenhaft ausgebildete Schichtaufbau der Nachweisvorrichtung mit der lichtundurchlässigen Reaktionsschicht dem Analyten ausgesetzt sein, und die lichtdurchlässige Nachweisschicht wird von ihrer Rückseite her in ihrer durch farbmarkierte Tracer verursachten Farbveränderung beobachtbar sein. Bei entsprechender Schichtdicke und der damit einhergehenden Erhöhung der Nachweisempfindlichkeit ist es jedoch auch günstig, die Schichtstruktur von einer der Stirnseiten aus zu beobachten, wobei wiederum die lichtundurchlässige Reaktionsschicht während des Meßvorgangs unverändert bleibt, die lichtdurchlässige Nachweisschicht jedoch, von ihrer Stirnseite her betrachtet, ihre Farbveränderung sichtbar werden läßt. Zur Verbesserung der Beobachtung kann es dabei sinnvoll sein, die Stirnfläche der Nachweisschicht mit einer optischen Sammellinse zu versehen, oder die Stirnfläche selbst als solche auszubilden, damit nicht nur die Färbung selbst besser erkennbar wird, sondern daß auch die Blickrichtung abweichend von der Normalen zur Stirnfläche erfolgen kann.

Um eine einfache, gerätemäßige optische Auswertemöglichkeit zu erhalten, kann man die eine Stirnfläche der Nachweisschicht mit einer Lichtquelle bestrahlen, und gegenüber der anderen Stirnfläche, die gegebenenfalls mit einer Zylinderlinse oder sonstigen geeigneten optischen Hilfsmitteln versehen ist, einen für bestimmte, im Zusammenhang mit dem Nachweis passende wellenlängen empfindlichen Detektor anbringen, so daß das empfangene Lichtsignal ein Maß für die angefallene Tracerkonzentration und somit für den Analyten ist. Diese Auswertung ist besonders dann vorzunehmen, wenn die Tracer nicht mit einem im sichtbaren Licht erkennbaren Markierungselement versehen sind.

Eine zweckmäßige Ausbildung der Vorrichtung als ein Monitor zur Überwachung von gasförmigen oder flüssigen Schadstoffen wird dadurch erreicht, daß der Träger in einem Gehäuse aufgenommen ist, welches einen Einlaßdeckel und einen Auslaßdeckel aufweist, die jeweils einen für den Durchtritt der zu untersuchenden Probe vorgesehenen Einlaß und Auslaß besitzen, zwischen denen der Träger eingespannt ist, welcher einen Schichtaufbau derart aufweist, daß vom Einlaß ausgehend zunächst die Reaktionsschicht und daran anschließend die Nachweisschicht in Diffusionskontakt vorliegt.

Ein derartiger Monitor ist geeignet, auf einfache weise eine passive oder auch aktive Probenahme durchzuführen. An der Vorderseite des Gehäuses befindet sich eine Öffnung oder auch ein Stutzen, die den Eintritt von Gas oder Flüssigkeit ermöglichen. Zur Durchführung einer aktiven Probenahme kann an der Rückseite des Gehäuses an den vorgesehenen Auslaß, welcher in Form eines Stutzens ausgebildet sein kann, eine Pumpe angeschlossen werden. Die Pumpe saugt aktiv das Gas oder die Flüssigkeit durch den Einlaß und durch den Träger, wo der Analyt an der gewünschten Reaktion teilnimmt. An dem Auslaß kann man ebenfalls einen von ihm abzweigenden Absaugestutzen vorsehen, durch den entweder eine flüssige Probe, oder bei einer gasförmigen Probe eine eventuell notwendige Waschlösung durch den Träger gesaugt und abgefangen werden kann. Der Träger kann als ein Membranfilter ausgebildet sein, bei dem kommerziell erhältliche Membranen geeigneter Porengröße von den Herstellern z.B. Millipore, Pall oder Satorius gewählt werden. In der Reaktionsschicht ist der für den Analyten spezifische Antikörper kovalent gebunden und z.B. mit einem Farbträger markiert. Der so gebildete Tracer/ Antikörper-Komplex geht mit dem Analyten eine Verdrängungsreaktion ein, durch die der Tracer durch den Analyten ersetzt wird. Der markierte Tracer diffundiert in die durchsichtige Nachweisschicht und reichert sich dort an. Bei einem monovalenten Analyten ergibt sich eine direkte Korrelation an verdrängtem Analyt zu dem Analyt in der gemessenen Probe.

Zweckmäßigerweise kann der Reaktionsschicht eine Sammelschicht vorgeschaltet sein, in welcher der Analyt zunächst gesammelt wird, bevor er mit den Tracern in der Reaktionsschicht eine immunologische Reaktion eingeht.

Um bei einer Verfärbung der Nachweisschicht durch die freien, nicht gebundenen farbmarkierten Tracer einen mit dem bloßen Auge erkennbaren Farbvergleich durchführen zu können, ist es günstig, auf der Nachweisschicht mehrere, unterschiedlich farbig ausgefüllte Markierungsbereiche aufzubringen. Diejenige Verfärbung, welche dem entsprechend farbig ausgefüllten Markierungsbereich entspricht, stellt ein Maß für die dem zugehörigen Markierungsbereich zugeordnete Konzentration oder Dosierung des Analyten dar.

Zur einfachen Handhabung ist es vorteilhaft, die beiden Deckel durch eine an ihren Längsseiten angebrachte Rastung gegeneinander zu verriegeln, so daß der Träger zwischen den beiden Deckeln eingeklemmt ist. Es kann ebenfalls günstig sein, die beiden Deckel mit einem Gelenkscharnier zu versehen und sie rastend ineinander zu klappen. Andere Befestigungsmöglichkeiten bestehen bei einer runden Ausführungsform der Deckel in einem Schraubgewinde oder einem Bajonettverschluß, durch welche beide Deckel aneinandergepreßt und der Träger zwischen ihnen eingeklemmt wird.

Zur Durchführung einer Messung wird beispielsweise an den Auslaß eine Pumpe mit vorgegebener Leistung angeschlossen, welche nach einer gewissen Zeitspanne eine definierte Menge von Gas oder Luft durch die Filtervorrichtung saugt. Dabei werden zu untersuchende Schadstoffe als Analyt auf der Sammelschicht zurückgehalten. Nach dem Messen wird die Sammelschicht mit einer bestimmten Menge wäßriger/ organisch-wäßriger Pufferlösung durchspült. Dabei kann die Flüssigkeit aktiv, wie zuvor die Luftprobe, mit Hilfe der Pumpe durch den Träger gesaugt werden, oder aber sie tropft passiv durch den Träger hindurch. Die dabei durchtropfende bzw. durchgesaugte Flüssigkeit wird über einen Absaugstutzen am Auslaß in eine Auffangflasche abgesaugt. Die gepufferte Lösung sorgt dafür, daß die konzentrierten Analyte von der Sammelschicht durch diese hindurch in die Reaktionsschicht gelangen. Gleichzeitig wird dabei die Reaktionsschicht getränkt und die gebundenen, markierten Antikörper/Antikörper-Tracer-Komplexe rehydratisiert. In diesem Zustand können Analyt und Antikörper in Verbindung treten und zu einer Verdrängungs- oder kompetitiven Reaktion führen. Zur Auswertung werden die Gehäusedeckel geöffnet, beispielsweise durch Aufdrehen oder Aufklappen, so daß der Träger sichtbar wird und die mehr oder weniger verfärbte Nachweisschicht durch Vergleich mit den farbig ausgefüllten Markierungsbereichen ausgewertet.

Mit der Nachweisvorrichtung zur immunologischen Bestimmung von Schadstoffen können als Analyte Insektizide (Pyrethroide, Triazin) Sprengstoffe (TNT) und Drogen sowie hochgiftige Substanzen (Dioxine) als sogenannte monovalente Analyte nachgewiesen werden, wobei darüberhinaus auch Bakterien wie E. coli K-12 zur überwachung von Gentechnologie-Labors, Pseudomonaden in Lüftungen von Krankenhäusern, Legionellen in Klimaanlagen nachgewiesen werden können. Die dazu geeigneten Antikörper lassen sich nach bekannten Verfahren herstellen, wie sie z.B. in Peters, J. H. und Baumgarten, H. (Herausgeber) "Monoklonale Antikörper, Herstellung und Charakterisierung" (1985), Springer-Verlag, Berlin, Germany, oder auch in Harlow, E. und Lane, D. (Herausgeber), "Antibodies, a laboratory manual" (1988), Cold Spring Harbor Lab., Cold Spring Harbor, USA, beschrieben sind.

Ein Ausführungsbeispiel der Erfindung wird anhand der schematischen Zeichnung dargestellt und im folgenden näher erläutert.

Es zeigen:

Fig. 1 einen Schnitt durch den schichtweisen Aufbau des Trägers mit symbolhafter Darstellung von Analyt, Tracer und Antikörper,

Fig. 2 den Schnitt nach Figur 1 während der Diffusion der verdrängten Tracer in der Nachweisschicht,

Fig. 3 die Ansicht des geschichteten Trägers von seiner Stirnseite aus.

Fig. 4 die perspektivische Ansicht einer Vorrichtung in Explosionsdarstellung

Das im folgenden geschilderte Beispiel wird anhand einer Verdrängungsreaktion erläutert. Wird eine Konkurrenzreaktion (kompetitiver Assay) beobachtet, laufen die einzelnen Reaktionsschritte, die zum Nachweis führen, mit dem beschriebenen Träger analog ab.

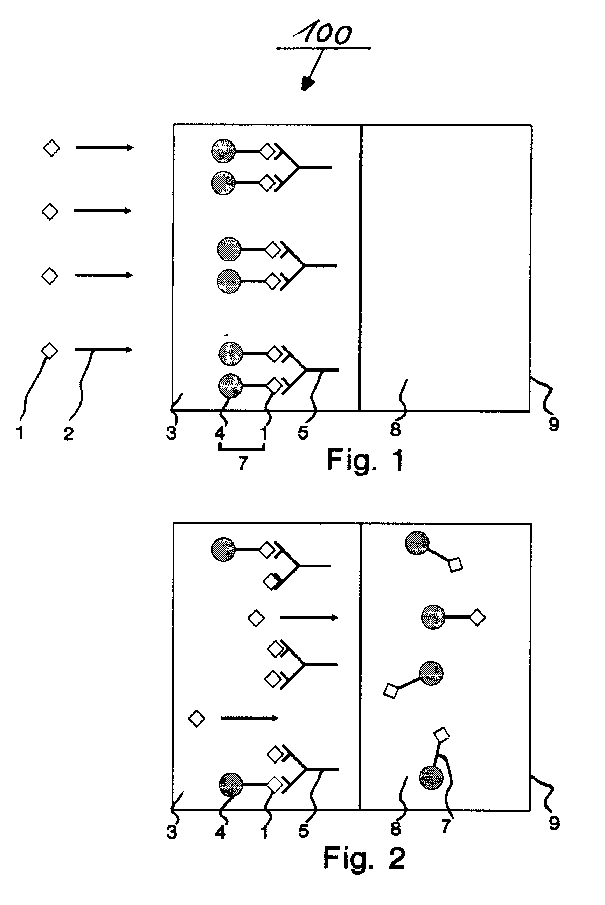

Der in Figur 1 dargestellte Träger (100) besteht aus zwei Teilbereichen in Form von Schichten, die entweder in selbsttragender Ausführungsform hergestellt sind, oder in einem nicht dargestellten Rahmen eingespannt und aufgenommen sind. Die in der zu untersuchenden Umgebung befindlichen Analyten (1) (als offene Rhomben dargestellt), bewegen sich entsprechend der dargestellten Richtungspfeile (2) auf die Oberfläche einer lichtundurchlässigen Reaktionsschicht (3), in der verteilt sich der Tracer/ Antikörper-Komplex (5, 7) befindet. Die Antikörper (5) (symbolisch dargestellt durch eine Y-Form) sind mit ihrem einen Zweig in der ersten lichtundurchlässigen Schicht (3) immobilisiert, so daß die gabelförmigen Zweige des Antikörpers (5) dem Analyten ausgesetzt sind. Der mit einem Farbstoff (4) markierte Analyt (1) wird als Tracer (7) bezeichnet. An die Reaktionsschicht (3) schließt sich eine lichtdurchlässige Nachweisschicht (8) an. Die Schichten (3, 8) sind in Diffusionskontakt miteinander verbunden.

Die Analyten (1) treffen auf die Reaktionsschicht (3), diffundieren in sie hinein (Fig. 2), verdrängen die Tracer (7) von ihrem Bindungsplatz am Antikörper (5) und nehmen deren Platz ein. Die verdrängten Tracer (7) diffundieren durch die Reaktionsschicht (3) in die Nachweisschicht (8), durch die sie von der Rückseite (9) der Nachweisschicht (8) wegen ihres an sie gekoppelten Farbstoffs (4) sichtbar werden.

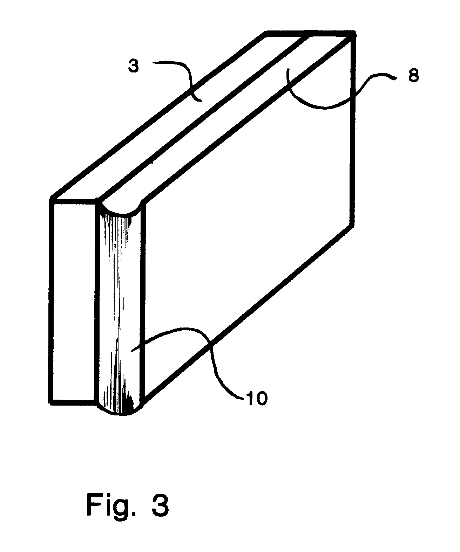

Der in Figur 3 dargestellte Schichtaufbau entspricht in wesentlichen Teilen demjenigen der Figur 1. Die lichtundurchlässige Reaktionsschicht (3) und die lichtdurchlässige Nachweisschicht (8) sind in direktem Diffusionskontakt aneinander gelegt. Die dem nicht dargestellten Beobachter zugewandte Stirnfläche der Nachweisschicht (8) ist in Form einer Zylinderlinse (10) ausgebildet.

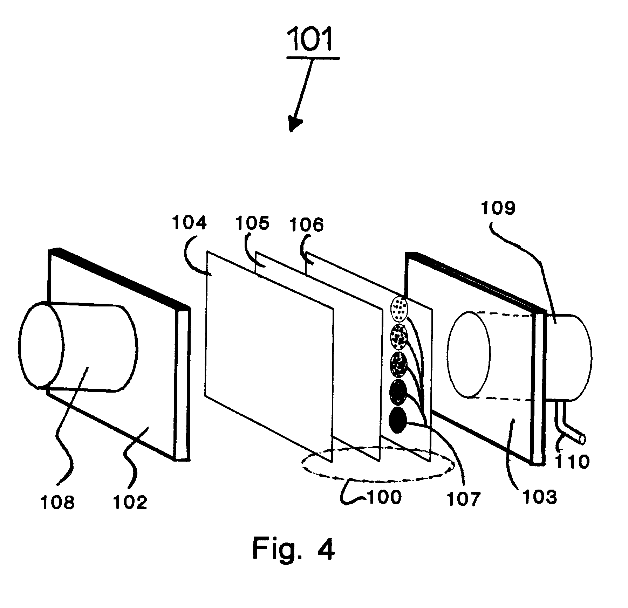

Die Figur 4 zeigt eine perspektivische Explosionsdarstellung der Vorrichtung, in welcher der Träger (100) zwischen einem Einlaßdeckel (102) und einem Auslaßdeckel (103) eingespannt ist. Die Deckel (102, 103) besitzen an ihrem Rand eine nicht dargestellte Einrastung, wodurch sie in zusammengedrücktem Zustand den Träger (100) zwischen sich einklemmen. Der Träger (100) ist zeichnungsmäßig in drei Schichten dargestellt, die jedoch gegenständlich sowohl in einer dreischichtigen Ausführungsform als auch in einer einzigen Membran durch unterschiedlich gestaltete Membranschichtbereiche aufgeteilt sein können. Zum Einlaßdeckel (102) hin gerichtet befindet sich als erste Schicht eine Sammelschicht (104), der sich in Diffusionskontakt eine mittlere Reaktionsschicht (105) anschließt. Die Schichtfolge ist abgeschlossen mit einer Nachweisschicht (106), welche längs eines ihrer Ränder kreisförmige Markierungsbereiche (107) aufweist. In der Figur sind fünf solcher Bereiche (107) dargestellt, die jeder mit einer unterschiedlichen Intensität gleicher Färbung ausgestattet ist. Je nach erforderlicher Auflösungsgenauigkeit sind mehr oder weniger solcher Markierungsbereiche (107) vorzusehen. Der Einlaßdeckel ist mit einem Einlaß (108), und der Auslaßdeckel (103) mit einem Auslaß (109) versehen, wobei der Einlaß den Zugang der Nachweisprobe (flüssig oder gasförmig) oder auch einer für den Nachweis erforderlichen Pufferlösung vorgesehen ist, und an den Auslaß (109) eine nicht dargestellte Saugpumpe angeschlossen werden kann. Zur Absaugung überschüssiger Flüssigkeitsmengen dient ein Absaugestutzen (110).

**Patentansprüche**

1. Vorrichtung zur quantitativen Analyse von Schadstoffen oder schadstoffhaltigen Aerosolen mit Hilfe einer immunologischen Reaktion, bei der der nachzuweisende Schadstoff (Analyt) mit einem Antikörper in Zusammenwirkung mit einem markierten Analyten (Tracer) zu einer immunologischen Reaktion gebracht wird, dadurch gekennzeichnet, daß die Antikörper (5) zusammen mit den markierten Tracern (7) in einem dem Analyten (1) zugänglichen und für diesen permeablen, undurchsichtigen Teilbereich (3) des Trägers (100) vorliegen, durch welche die nach erfolgter immunologischer Reaktion in dem undurchsichtigen Teilbereich (3) verbleibenden freien, nicht an einen Antikörper (5) gebundenen Tracer (7) in einen transparenten Teilbereich (8) des Trägers (100) diffundieren, so daß das Fortschreiten der Tracerdiffusion an der Außenfläche (9, 10) des transparenten Teilbereichs (8) des Trägers (100) durch dessen Verfärbung erkennbar ist.

2. Vorrichtung nach Anspruch 1, dadurch gekennzeichnet, daß der markierte Tracer (7) an den Antikörper (5) gebunden und einen Tracer/Antikörper-Komplex bildend in dem Teilbereich (3) vorliegt.

3. Vorrichtung nach Anspruch 1, dadurch gekennzeichnet, daß der markierte Tracer (7) reversibel, und der Antikörper (5) kovalent gebunden jeweils getrennt für sich in dem Teilbereich (3) vorliegen.

4. Vorrichtung nach einem der Ansprüche 1 bis 3, dadurch gekennzeichnet, daß der Tracer (7) mit einem sichtbaren Farbstoff (4) markiert ist.

5. Vorrichtung nach einem der Ansprüche 1 bis 4, dadurch gekennzeichnet, daß der Träger (100) zwei Schichten als Teilbereiche aufweist, von denen die eine als lichtundurchlässige Reaktionsschicht (3) mit den an dieser immobilisierten Tracern (7) dem zu untersuchenden Analyt (1) ausgesetzt ist, an welche die andere, für die verbleibenden freien Tracer (7) permeable Nachweisschicht (8) im Diffusionskontakt anliegt.

6. Vorrichtung nach einem der Ansprüche 1 bis 5, dadurch gekennzeichnet, daß der Analyt mit einem Polymer versehen ist, der eine Vielzahl von Kopplungsstellen für dessen Markierung aufweist.

7. Vorrichtung nach einem der Ansprüche 1 bis 6, dadurch gekennzeichnet, daß die beiden Bereiche (3, 8) eine insgesamt voneinander verschiedene elektrische Ladung oder Polarität aufweisen, und daß die Tracer (7) durch ihre Ladung oder Polarität eine Affinität zu der Nachweisschicht besitzen.

8. Vorrichtung nach einem der Ansprüche 1 bis 7, dadurch gekennzeichnet, daß der Träger (100) in einem Gehäuse (101) aufgenommen ist, welches einen Einlaßdeckel (102) und einen Auslaßdeckel (103) aufweist, die jeweils einen für den Durchtritt der zu untersuchenden Probe vorgesehenen Einlaß (108) und Auslaß (109) besitzen, zwischen denen der Träger (100) eingespannt ist, welcher einen Schichtaufbau derart aufweist, daß vom Einlaß (108) ausgehend zunächst die Reaktionsschicht (105) und daran anschließend die Nachweisschicht (106) in Diffusionskontakt vorliegt.

9. Vorrichtung nach einem der Ansprüche 1 bis 8, dadurch gekennzeichnet, daß der Reaktionsschicht (105) eine Sammelschicht (104) vorgeschaltet ist, in welcher der Analyt aus der Probe aufgefangen wird und für die Reaktion mit dem in der Reaktionsschicht (105) immobilisierten Tracer (7) aufbewahrt ist.

10. Vorrichtung nach einem der Ansprüche 1 bis 9, dadurch gekennzeichnet, daß auf der Nachweisschicht (106) mehrere, unterschiedlich farbig ausgefüllte Markierungsbereiche (107) aufgebracht sind, die nach erfolgter Messung als Farbvergleichsstandard für den Verfärbungsgrad der durch die freien, farbmarkierten Tracer (7) verfärbten Nachweisschicht (106) herangezogen werden.

11. Vorrichtung nach einem der Ansprüche 8 bis 10, dadurch gekennzeichnet, daß die beiden Deckel (102, 103) durch eine an ihren Längsseiten angebrachte Rastung gegeneinander verriegelbar sind, und daß der Träger (100) in verriegeltem Zustand zwischen beiden eingeklemmt ist.

Fig. 1

Fig. 2

Fig. 3

101

104   105   106   109

108   102   100   107   103   110

Fig. 4